## Europäisches Patentamt
## European Patent Office
## Office européen des brevets

(11) Veröffentlichungsnummer: **0 005 574**
**B1**

(12) **EUROPÄISCHE PATENTSCHRIFT**

(45) Veröffentlichungstag der Patentschrift:
**16.09.81**

(21) Anmeldenummer: **79200234.7**

(22) Anmeldetag: **12.05.79**

(51) Int. Cl.³: **C 07 C 29/80,** C 07 C 63/08,
C 07 C 51/42, C 07 C 27/02,
C 07 C 29/86, C 07 C 51/48,
C 07 C 29/09, C 07 C 51/41,
C 07 C 33/22

(54) Verfahren zur Herstellung eines Alkalimetallbenzoats neben einem Benzylalkohol.

(30) Priorität: **19.05.78 NL 7805415**

(43) Veröffentlichungstag der Anmeldung:
**28.11.79 Patentblatt 79/24**

(45) Bekanntmachung des Hinweises auf die Patenterteilung:
**16.09.81 Patentblatt 81/37**

(84) Benannte Vertragsstaaten:
**BE DE FR GB IT NL**

(56) Entgegenhaltungen:
**DE-A-2 758 391**
**NL-C-106 100**
**US-A-1 601 509**
**US-A-3 907 877**

(73) Patentinhaber: **STAMICARBON B.V., Postbus 10,
NL-6160 MC Geleen (NL)**

(72) Erfinder: **Jongsma, Cornelis, Drossaertweide 1,
NL-6438 HS Oirsbeek (NL)**

(74) Vertreter: **Hoogstraten, Willem Cornelis Roeland et al,
OCTROOIBUREAU DSM Postbus 9, NL-6160 MA Geleen
(NL)**

Verfahren zur Herstellung eines Alkalimetallbenzoats neben einem Benzylalkohol

Die vorliegende Erfindung betrifft ein Verfahren zur Herstellung eines Alkalimetallbenzoats, insbesondere Natriumbenzoat, neben einem Benzylalkohol durch Hydrolyse eines Benzylbenzoats.

Aufgabe der Erfindung ist insbesondere eine geeignete Methode zur Verarbeitung eines verunreinigten Benzylbenzoats, das bei der Oxidation einer Monoalkylbenzolverbindung mit einem molekularen Sauerstoff enthaltenden Gas anfällt. Die Oxidation vor Toluol wird in grosstechnischem Massstab ausgeführt, und das Benzylbenzoat, das dabei anfallen kann, ist das unsubstituierte Benzylbenzoat, d. h., dass beide Benzolkerne des Benzylbenzoats nicht substituiert sind. Wegen dieser technischen Bedeutung wird die Erfindung hauptsächlich anhand der Oxidation von Toluol und des dabei anfallenden verunreinigten, unsubstituierten Benzylbenzoats erläutert. Die Erfindung ist jedoch auch bei verunreinigten substituierten Benzylbenzoaten anwendbar, die z. B. bei der Oxidation anderer Alkylbenzolverbindungen anfallen können, insbesondere bei der Oxidation solcher Alkylbenzolverbindungen, deren Alkylgruppe 1–4 Kohlenstoffatome enthält und die ggf. am Benzolkern noch nicht störende Substituenten, wie Halogen-, Nitrooder Sulfonsäure-Substituenten, enthalten können.

Beispiele sind Äthylbenzol, p-Chlortoluol, p-Nitrotoluol und p-Toluolsulfonsäure.

Die Oxidationsreaktion kann entweder in der flüssigen Phase mit z. B. einem im Reaktionsmittel löslichen Kobalt- und/oder Mangansalz als Katalysator oder in der Gasphase mit z. B. einem Katalysator auf Basis eines Oxids von Vanadium oder einem anderen Übergangsmetall statfinden (Stanford Research Institute (SRI)-Berichte Nr. 7 (1965), 29; Nr. 7A (1968), 241; Nr. 7B (1976), 53).

Man kann aus dem Reaktionsgemisch, das Benzoesäure, Benzylbenzoat, andere höher als Benzoesäure siedende Produkte, die hier unter dem Namen Teerrückstand zusammengefasst werden, unumgesetztes Toluol und niedriger als Benzoesäure siedende Nebenprodukte, wie Benzylalkohol und Benzaldehyd, enthält, die Benzoesäure, vollständig oder teilweise, zusammen mit allen niedriger siedenden Produkten abdestillieren und das Destillat weiter auf reine Benzoesäure aufarbeiten, so dass verunreinigtes Benzylbenzoat in Form eines Teeres, der eventuell noch Benzoesäure enthalten kann, übrigbleibt. Dieser Rückstand kann als Ausgangsmaterial für das erfindungsgemässe Verfahren dienen.

Verunreinigtes Benzylbenzoat, das ebenfalls als Ausgangsmaterial für das erfindungsgemässe Verfahren dienen kann, kann auch erhalten werden, wenn man obengenanntes Destillat in Gegenwart oder Abwesenheit eines Veresterungs/ Umesterungskatalysators, wie Schwefelsäure, Phosphorsäure, Bortrifluorid oder Zinkacetat, erhitzt, um den vorhandenen Benzylalkohol und/ oder leichte Benzylester, wie Benzylformiat und Benzylacetat, zu Benzylbenzoat zu verestern bzw. umzuestern. Störende Verunreinigungen entstehen insbesondere dann, wenn das Gemisch während dieser esterbildenden Reaktion noch Benzaldehyd enthält.

Es ist ausserordentlich schwierig, aus derart verunreinigtem Benzylbenzoat nützliche Produkte zu gewinnen. Zwar kann man Benzylbenzoat aus dem Teerrückstand abdestillieren, es ist aber fast nicht möglich, auf diese Weise ein reines Benzylbenzoatprodukt zu erhalten. Anmelderin hat gefunden, dass das im Teer enthaltene Fluorenon besondere Schwierigkeiten verursacht. Fluorenon lässt sich nämlich fast nicht von Benzylbenzoat trennen. Neben Fluorenon können aber auch andere, nicht identifizierte Verbindungen eine Rolle spielen. Es ist zwar möglich, das Benzylbenzoat, ggf. nachdem es vom Teerrückstand abdestilliert worden ist, mit z. B. wässeriger Natronlauge zu Benzylalkohol und Natriumbenzoat zu hydrolysieren, das Natriumbenzoat lässt sich aber sehr schwer in reiner Form gewinnen, da gewisse Verunreinigungen sich hartnäckig mit dem Natriumbenzoat vereinigen. Natriumbenzoat ist in reiner Form ein wertvoller Stoff, an dem ein deutlicher Bedarf besteht. Es ist u. a. ein wichtiges Konservierungsmittel.

Nach der vorliegenden Erfindung stellt man ein Alkalimetallbenzoat neben einem Benzylalkohol aus einem verunreinigten Benzylbenzoat, das bei der Oxidation einer Monoalkylbenzolverbindung mit einem molekularen Sauerstoff enthaltenden Gas erhalten wird, dadurch her, dass man das verunreinigte Benzylbenzoat in Gegenwart eines basischen Stoffes destilliert und das anfallende Destillat mit einer Alkalimetallbase hydrolisiert.

Auf diese Weise kann aus dem verunreinigten Benzylbenzoat ein reines Alkalimetallbenzoat erhalten werden. Weiterhin kann man aus dem hydrolisierten Gemisch Benzylalkohol in reiner Form gewinnen. Benzylalkohol ist ein nützliches Produkt, das in der Geruch- und Geschmacksstoffindustrie angewendet wird. Mit dem erfindungsgemässen Verfahren wird somit erreicht, dass ein bisher unbrauchbares Abfallprodukt in zwei nützliche Stoffe umgesetzt wird.

Als basischen Stoff bei der Destillation des Benzylbenzoats kann man z. B. ein Oxid, Hydroxid oder Carbonat eines Alkali- oder Erdalkalimetalls, Ammoniak, ein Amin, ein ggf. am Stickstoffatom substituiertes Ammoniumsalz oder ein Amid benutzen. Bevorzugt werden (festes) Natriumhydroxid, Natriumcarbonat, Kaliumhydroxid, Kaliumcarbonat, Calciumhydroxid, Calciumoxid, Ammoniak und insbesondere ein Ammoniumcarbonat. Als Amin verwendet man vorzugsweise ein aliphatisches und vorzugsweise primäres Amin mit vorzugsweise 1–6 Kohlenstoffatomen je Alkylgruppe, z. B. Methylamin, Äthylamin, Propylamin, Isopropylamin, Butylamin, Isobutylamin, Cyclopentylamin oder Cyclohexylamin. Auch Salze von Ammoniak und von den vorgenannten Aminen, d. h. substituierte oder nicht substituierte Ammoniumverbindungen, die sich unter den Destillationsbedingungen zersetzen können, z. B. die Wasserstoffcarbonate, Carbonate, Formiate, Acetate und Benzoate, können mit Vorteil angewendet werden. Als Amid kann man z. B. ein Carbonsäureamid verwenden, vorzugsweise Harnstoff. Die benutzte Menge basischer Stoff beträgt z. B. 0,01 bis 10 Gew.-% und vorzugsweise 0,5 bis 5 Gew.-%, bezogen auf das unreine Benzylbenzoat.

Die Destillation kann bei ungefähr atmosphärischem oder etwas höherem Druck ausgeführt werden, z. B. bis 200 kPa, vorzugsweise jedoch bei vermindertem Druck, z. B. 0,1 bis 10 kPa. Die Temperatur beträgt z. B. 80 bis 240 °C.

Die Hydrolyse des Destillats kann mit einer basischen Lösung, insbesondere mit wässeriger Natronlauge oder eventuell mit einer Sodalösung, ausgeführt werden. Auch könnte z. B. Kalilauge oder Kaliumcarbonat oder eine andere Alkalimetallbase verwendet werden. Es besteht aber vor allem ein Bedürfnis an Natriumbenzoat als Reaktionsprodukt, so dass die Hydrolyse vorzugsweise mit einer basischen Natriumverbindung ausgeführt wird. Die Temperatur kann bei der Hydrolysenreaktion z. B. zwischen 30 und 200 °C liegen. Der Druck ist nicht kritisch und liegt aus praktischen Gründen vorzugsweise zwischen 100 und 1000 kPa.

Nach der Hydrolyse kann der auf diese Weise erhaltene Benzylalkohol z. B. mittels Destillation, z. B. Dampfdestillation, abgetrennt werden. Das Alkalimetallbenzoat kann durch Kristallisation aus der wässerigen Lösung in fester Form gewonnen werden.

Meistens enthält das Hydrolysengemisch neben dem Benzylalkohol und dem Alkalimetallbenzoat noch störende organische Verunreinigungen. In diesem Fall extrahiert man das Hydrolysenreaktionsgemisch vorzugsweise mit einem organischen Extrahiermittel, das mit dem Hydrolysenreaktionsgemisch zur Flüssigphasentrennung führt.

Dabei gehen der Benzylalkohol und die organischen Verunreinigungen in die organische Phase über, während die wässerige Phase aus einer nahezu reinen Alkalimetallbenzoatlösung besteht, aus der auf einfache Weise reines, festes Alkalimetallbenzoat gewonnen werden kann. Aus der organischen Phase kann man durch Destillation reinen Benzylalkohol gewinnen und das Extrahiermittel zurückgewinnen.

Beispiele für geeignete organische Extrahiermittel sind aliphatische, aromatische und gemischt aliphatisch-aromatische Kohlenwasserstoffe mit vorzugsweise maximal 12 Kohlenstoffatomen je Molekül, Äther, Ester und halogenierte und insbesondere chlorierte oder bromierte Kohlenwasserstoffe mit normalen Siedepunkten von vorzugsweise nicht mehr als 250 °C. Spezifische Beispiele sind Benzin, Heptan, Benzol, Toluol, die Xylole, Diisopropyläther, Amylacetat, Äthylbenzoat, Chloroform, 1,2-Dichloräthylen, 1,1,1,-Trichloräthan und 1,2-Dibromäthan. Besonders bevorzugt wird Toluol, da dies in grossen Mengen in einer Toluoloxidationsanlage vorhanden ist und eine gute Trennung garantiert.

Nach einer geeigneten Ausführungsform des erfindungsgemässen Verfahrens führt man das Produktgemisch der Hydrolysenreaktion einer Extraktionskolonne zu, in die man auch das Extrahiermittel einleitet.

Es ist auch möglich, dass das Extrahiermittel bereits bei der Hydrolysenreaktion vorhanden ist. Selbstverständlich ist es dann empfehlenswert, ein Extrahiermittel zu wählen, das unter den betreffenden Reaktionsbedingungen inert ist. Nach der Reaktion trennt man das Produktgemisch dann in eine den Benzylalkohol, organische Verunreinigungen und das Extrahiermittel enthaltende organische Schicht und eine das Alkalimetallbenzoat enthaltende wässerige Schicht.

Man kann die Hydrolysenreaktion dabei in einer Extraktionskolonne stattfinden lassen, durch die das in Gegenwart eines basischen Stoffes destillierte Benzylbenzoat und die basische Lösung im Gegenstrom mit dem Extrahiermittel hindurchgeleitet werden.

Die Erfindung wird anhand der nachstehenden, nicht einschränkenden Beispiele näher erläutert.

Beispiel I

Das flüssige Reaktionsprodukt, das bei der Oxidation von Toluol in der flüssigen Phase mit Luft in Gegenwart von Kobaltacetat als Katalysator (Toluolumsetzungsgrad ca. 20 Gew.-%) anfällt, wird destilliert, bis die Benzoesäure und niedriger siedenden Bestandteile nahezu vollständig aus dem Reaktionsprodukt entfernt sind. Der Rückstand wird bei einer Temperatur von 230 °C und einem Druck von 7 kPa einer Dünnschichtverdampfung unterzogen. Das bei der Dünnschichtverdampfung anfallende Destillat, ein Gemisch von Benzylbenzoat, etwas Benzoesäure und Teer, wird bei einer Temperatur von 140 °C und einem Druck von 0,8 kPa nochmals destilliert, diesmal in Gegenwart von 3 Gew.-% festem Ammoniumwasserstoffcarbonat, bezogen auf das bei der Dünnschichtverdampfung erhaltene Destillat.

Dem Destillat werden 20 Gew.-% bezogen auf das Destillat, Natriumhydroxid in Form von wässeriger Natronlauge (14 Gew.-% NaOH) beigeben, und das Ganze wird 30 Minuten unter Rückfluss bei einer Temperatur von 100 °C gerührt. Nahezu alles Benzylbenzoat ist nun hydrolisiert.

Nach Abkühlung wird das Reaktionsgemisch viermal mit gleichen Mengen Toluol (die Gesamtmenge entspricht zweimal dem Gewicht des vorhandenen Wassers) extrahiert. Die auf diese Weise erhaltenen Toluolschichten werden zusammengefügt und mit Wasser (10 Gew.-%, bezogen auf die vorhandene Toluolmenge) gewaschen. Nach Trennung der Schichten wird das Waschwaser mit oben genannter Wasserschicht zusammengefügt.

Der kombinierten Wasserschicht wird zur Neutralisierung des noch vorhandenen Natriumhydroxids Benzoesäure beigegeben. Anschliessend wird das Reaktionsgemisch mit Dampf destilliert, wonach durch Entfernung des Wassers festes Natriumbenzoat in nahezu quantitativer Ausbeute abgetrennt wird.

Eine Lösung des auf diese Weise erhaltenen Natriumbenzoats genügt den Anforderungen des Farbtests der niederländischen Pharmakopöe. Das Natriumbenzoat genügt auch den Anforderungen der Schwefelsäureprüfung DAB 7 der deutschen Pharmakopöe.

Aus der gewaschenen Toluolschicht werden das Toluol und das vorhandene Wasser bei atmosphärischem Druck abdestilliert. Anschliessend wird der Rückstand bei 70 °C und in 1 kPa destilliert. Der Kopfstrom dieser Destillation besteht aus nahezu reinem Benzylalkohol (Reinheit mehr als 99 Gew.-%). Die Ausbeute an Benzylalkohol nach der Destillation im Vergleich zum Benzylbenzoat beträgt mehr als 90%.

Beispiel II

Beispiel I wird wiederholt, wobei statt Ammoniumwasserstoffcarbonat festes Natriumhydroxid in einer Menge von 3 Gew.-%, bezogen auf das bei der Dünnschichtverdampfung erhaltenem Destillat, verwendet wird.

Es fällt wieder nahezu quantitativer Ausbeute Natriumbenzoat an, das den in den genannten Reinheitsprüfungen erwähnten Anforderungen genügt, neben nahezu reinem Benzylalkohol mit einer Ausbeute von mehr als 90%, bezogen auf Benzylbenzoat.

**Patentansprüche**

1. Verfahren zur Herstellung eines Alkalimetallbenzoats neben einem Benzylalkohol aus einem verunreinigten Benzylbenzoat, das bei der Oxidation einer Monoalkylbenzolverbindung mit einem molekularen Sauerstoff enthaltenden Gas anfällt, dadurch gekennzeichnet, dass man das verunreinigte Benzylbenzoat in Gegenwart eines basischen Stoffes destilliert und das anfallende Destillat mit einer Alkalimetallbase hydrolysiert.

2. Verfahren nach Anspruch 1, dadurch gekennzeichnet, dass der bei der Destillation benutzte basische Stoff ein Oxid, Hydroxid oder Carbonat eines Alkali- oder Erdalkalimetalls, Ammoniak, ein Amin, ein ggf. am Stickstoffatom substituiertes Ammoniumsalz oder ein Amid ist.

3. Verfahren nach Anspruch 2, dadurch gekennzeichnet, dass der bei der Destillation benutzte basische Stoff ein Ammoniumcarbonat ist.

4. Verfahren nach Anspruch 2, dadurch gekennzeichnet, dass der bei der Destillation benutzte basische Stoff Natriumhydroxid ist.

5. Verfahren nach Anspruch 2, dadurch gekennzeichnet, dass der bei der Destillation benutzte basische Stoff ein primäres aliphatisches Amin mit 1–6 Kohlenstoffatomen ist.

6. Verfahren nach Anspruch 2, dadurch gekennzeichnet, dass der bei der Destillation benutzte basische Stoff Harnstoff ist.

7. Verfahren nach einem der Ansprüche 1–6, dadurch gekennzeichnet, dass die Hydrolysenreaktion mit Natriumhydroxid oder Natriumcarbonat ausgeführt und aus dem Produktgemisch das Natriumbenzoat gewonnen wird.

8. Verfahren nach Anspruch 7, dadurch gekennzeichnet, dass das Produktgemisch mit einem organischen Extrahiermittel extrahiert und aus dem Extrakt der Benzylalkohol gewonnen wird.

9. Verfahren nach Anspruch 8, dadurch gekennzeichnet, dass als Extrahiermittel Toluol verwendet wird.

10. Verfahren nach Anspruch 8 oder 9, dadurch gekennzeichnet, dass die Hydrolysenreaktion in Gegenwart des Extrahiermittels ausgeführt wird.

**Claims**

1. Process for the preparation of an alkali-metal benzoate, in addition to a benzyl alcohol, from a contaminated benzyl benzoate obtained in the oxidation of a monoalkyl-benzene compound with a gas containing molecular oxygen, characterized in that the contaminated benzyl benzoate is distilled in the presence of a basic substance and the resulting distillate is hydrolyzed with an alkali-metal base.

2. Process according to claim 1, characterized in that the basic substance used in the distillation is an oxide, hydroxide or carbonate of an alkali or alkaline-earth metal, ammonia, an amine, an ammonium salt with or without a substituent at the nitrogen atom, or an amide.

3. Process according to claim 2, characterized in that the basic substance used in the distillation is an ammonium carbonate.

4. Process according to claim 2, characterized in that the basic substance used in the distillation is sodium hydroxide.

5. Process according to claim 2, characterized in that the basic substance used in the distillation is a primary aliphatic amine with 1–6 carbon atoms.

6. Process according to claim 2, characterized in that the basic substance used in the distillation is urea.

7. Process according to any one of the claims 1–6, characterized in that the hydrolysis reaction is effected with sodium hydroxide or sodium carbonate and the sodium benzoate is recovered from the product mixture.

8. Process according to claim 7, characterized in that the product mixture is extracted with an organic extraction agent and the benzyl alcohol is recovered from the extract.

9. Process according to claim 8, characterized in that the extraction agent used is toluene.

10. Process according to claim 8 or 9, characterized in that the hydrolysis reaction is effected in the presence of an extraction agent.

**Revendications**

1. Procédé de préparation d'un benzoate de métal alcalin à côté d'un alcool benzylique à partir d'un benzoate de benzyle souillé qui a été obtenu moyennant l'oxydation d'un composé de benzène mono-alcoylique à l'aide d'un gaz contenant de l'oxygène moléculaire, caractérisé en ce qu'on distille le benzoate de benzyle souillé en présence d'une matière basique et qu'on hydrolyse le distillat obtenu à l'aide d'une base de métaux alcalins.

2. Procédé selon la revendication 1, caractérisé en ce que la matière basique, utilisée pour la distillation, est un oxyde, un hydroxyde ou un carbonate d'un métal alcalin ou alcalino-terreux, de l'ammoniaque, une amine, un sel d'ammonium substitué ou non à l'atome d'azote ou un amide.

3. Procédé selon la revendication 2, caractérisé en ce que la matière basique, utilisée pour la distillation, est un carbonate d'ammonium.

4. Procédé selon la revendication 2, caractérisé en ce que la matière basique, utilisée pour la distillation, est de l'hydroxyde de sodium.

5. Procédé selon la revendication 2, caractérisé en ce que la matière basique, utilisée pour la distillation, est une amine primaire aliphatique avec 1–6 atomes de carbone.

6. Procédé selon la revendication 2, caractérisé en ce que la matière basique, utilisée pour la distillation, est de l'urée.

7. Procédé selon l'une des revendications 1 à 6, caractérisé en ce qu'on effectue la réaction d'hydrolyse à l'aide d'hydroxyde de sodium ou de carbonate de sodium et qu'on obtient le benzoate de sodium à partir du mélange de produit.

8. Procédé selon la revendication 7, caractérisé en ce qu'on extrait le mélange de produit à l'aide d'un agent d'extraction organique et qu'on obtient l'alcool benzylique à partir de l'extrait.

9. Procédé selon la revendication 8, caractérisé en ce qu'on utilise du toluène comme agent d'extraction.

10. Procédé selon l'une des revendications 8 à 9, caractérisé en ce qu'on effectue la réaction d'hydrolyse en présence de l'agent d'extraction.